Europäisches Patentamt

European Patent Office

Office européen des brevets

(19)

(11) Publication number: **0 379 295**
**A2**

(12) **EUROPEAN PATENT APPLICATION**

(21) Application number: 90300248.3

(22) Date of filing: **09.01.90**

(51) Int. Cl.5: **A61K 39/395, C07K 15/00,**
**A61K 37/64, A61K 39/00**

(30) Priority: **13.01.89 JP 6985/89**

(43) Date of publication of application:
**25.07.90 Bulletin 90/30**

(84) Designated Contracting States:
**AT BE CH DE DK ES FR GB GR IT LI LU NL SE**

(71) Applicant: **TAISHO PHARMACEUTICAL CO. LTD**
**24-1 Takata 3-chome Toshima-ku Tokyo 171(JP)**

(72) Inventor: **Katunuma, Nobuhiko**
**246-2, Myodocho-3-chome Tokushima-shi(JP)**

(74) Representative: **Lamb, John Baxter et al**
**MARKS & CLERK 57/60 Lincoln's Inn Fields London WC2A 3LS(GB)**

(54) Anti-tryptase antibody and composition for treatment of AIDS using the same.

(57) Anti-tryptase antibody capable of specifically reacting with tryptase significantly suppresses HIV infectivity. Pharmaceutical compositions comprising the anti-tryptase antibody are effective for the treatment of AIDS and tryptase is useful as a peptide vaccine for AIDS.

EP 0 379 295 A2

# ANTI-TRYPTASE ANTIBODY AND COMPOSITION FOR TREATMENT OF AIDS USING THE SAME

## BACKGROUND OF THE INVENTION

### FIELD OF THE INVENTION

The present invention relates to an anti-tryptase antibody and a pharmaceutical composition for the treatment of acquired immunodeficiency syndrome (AIDS) using the same. More specifically, the present invention relates to an anti-tryptase antibody which can bind to tryptase, one of trypsin type serine proteases, and to a pharmaceutical composition for the treatment of AIDS comprising the anti-tryptase antibody as an active ingredient, and to a peptide vaccine for AIDS.

### STATEMENT OF THE RELATED ART

Acquired immunodeficiency syndrome (AIDS) is a serious immunodeficiency induced by human immunodeficiency virus (HIV) and is now spreading worldwide. It has been desired to develop a drug for the treatment of AIDS or a vaccine thereof.

HIV, which is a causative agent of AIDS, is one of retroviruses having RNA as a genome. More specifically, human immunodeficiency virus type 1 (HIV-1) was isolated from lymphocytes of patients with lymphoma and human immunodeficiency virus type 2 (HIV-2) was isolated from patients with AIDS in Western Africa [Mireidlle et al., nature, 326, 662 (1987)].

It was revealed that HIV particles bind to CD4 antigens on the surface of helper T-cells which play the central role in immunoreaction and invade into the cells to kill helper T-cells, and this causes immunodeficiency. It has also been revealed that HIV infection is initiated by the binding of gp120, an envelope glycoprotein of HIV, to CD4 molecules on the surface of helper T-cells [Dalgleis et al., Nature, 312, 763 (1984); Klatzmann et al., Nature, 312, 767 (1984); McDougal et al., Science, 231, 382 (1986)]. Based on such mechanism of infection, an approach has been made using antibodies to gp120 as a drug to inhibit HIV infection [Robey et al., Proc. Natl. Acad. Sci. USA, 83, 7023 (1986)].

It was reported that the epitope of HIV neutralizing-monoclonal antibody was located between amino acids 308-331 of gp120 [Matsushita et al., J. Virol., 62, 2107 (1988)].

Furthermore, it was reported that among the monoclonal antibodies to various synthetic oligopeptides comprising of a part of amino acid sequence of gp120, only the monoclonal antibody to the synthetic oligopeptide corresponding to the amino acids 308 - 331 inhibited the HIV infection [Palker et al., Proc. Natl. Acad. Sci. USA, 85, 1932 (1988)]. It is known that the region corresponding to the amino acids 303 - 321 is not involved in the binding of gp120 to CD4 [Kowalski et al., Science, 237, 1351 (1987); Lasky et al., Coll, 50, 975 (1987)]. Therefore, it may be possible to speculate that even an antibody to the region which is not involved in the binding to CD4 could modify the tertiary structure of gp120 and inhibit the binding of gp120 to CD4. However, it was reported that an antibody to synthetic oligopeptide corresponding to the amino acids 504 - 518 in gp120 could not inhibit the syncytium formation while this antibody could bind to gp120 itself [Palker et al., Proc. Natl. Acad. Sci. USA, 85, 1932 (1988)].

From these facts, the regions corresponding to the amino acids 504 - 518, 303 - 321, and 308 - 331 can be recognized by the antibodies, but only the antibodies to the latter two regions can inhibit the HIV infection. Therefore, the latter two regions must play an important role in the binding of gp120 to CD4. This region is located in a variable region, in which difference of amino acid sequence is often found among clinically isolated HIV.

On the other hand, a new protease inhibitor, trypstatin, was recently isolated from rat mast cells and its primary structure was identified [Kido et al., J. Biol. Chem., 263, 18104 (1988); Kido et al., Cell Engineering, 7, 851 (1988); Kido et al., Metabolism, 25, extra volume, entitled "Highlight of Metabolic Disease", 187 (1988)]. Typstatin is a specific inhibitor of tryptase, which is a new trypsin type serine protease. Tryptase, having a molecular weight of 140,000, was isolated from granules of rat mast cells and consists of four subunits with each a molecular weight of 35,000 [Kido et al., Arch. Biochem. Biophys. 239, 436 (1985); Kido et al., Metabolism, 25, extra volume entitled "Highlight of Metabolic Disease", 187 (1988)].

It is known that tryptase catalyzes conversion from prothrombin into α-thrombin in the blood coagulation system and trypstatin inhibits this action, and that trypstatin is closely involved in the formation of allergic inflammation [Kido et al., Metabolism, 25, extra volume entitled "Highlight of Metabolic Disease", 187 (1988); Kido et al., Cell Engineering, 7, 851 (1988)].

The present inventor has paid his attention to the variable region of gp120 which will be closely involved in the binding of gp120 to CD4 molecule on the surface of helper T-cells, which is the initial step of HIV infection. Among the variable region, the inventor found a relatively well conserved sequence, that is, Gly-Pro-Gly-Arg-Ala-Phe. If this sequence was closely involved in the binding to CD4, a substance having a sequence similar to the aforesaid one would be expected to inhibit the binding of gp120 to CD4. As such substances, the present inventor has paid attention to trypstatin and made investigations on the inhibiting activity of the syncytia formation which was an evidence of HIV infection. It has thus been found that trypstatin was a potent inhibitor of syncytia formation and was highly effective as agents for the treatment of AIDS.

Therefore, it was suggested that tryptase-like enzyme which can be inhibited by trypstatin would be present on the surface of T-cells and this enzyme would be closely involved in HIV infection and hence, an antibody to tryptase would also be expected to have an inhibitory activity on syncytia formation. Thus, the present inventor has made extensive investigations on the inhibiting activity of the syncytia formation with the anti-tryptase antibody obtained by immunizing animal with tryptase. As a result, it has been found that the anti-tryptase antibody has a potent inhibitory activity of syncytia formation and is effective as a drug for the treatment of AIDS. It has also been found that tryptase itself can be used as a vaccine for AIDS. The present invention has thus been accomplished.

SUMMARY OF THE INVENTION

An object of the present invention is to provide an anti-tryptase antibody capable of specifically reacting with tryptase.

Another object of the present invention is to provide a pharmaceutical composition for the treatment of AIDS comprising the anti-tryptase antibody as an active ingredient.

A further object of the present invention is to provide a vaccine for AIDS.

In one aspect, the present invention is directed to an anti-tryptase antibody capable of specifically reacting with tryptase or a fragment thereof containing the antigen-binding site.

In another aspect, the present invention is directed to a pharmaceutical composition for the treatment of AIDS comprising anti-tryptase antibody as an active ingredient, which can specifically bind to tryptase or a fragment thereof containing the antigen-binding site.

In further aspect, the present invention is directed to a peptide vaccine for AIDS comprising tryptase.

DETAILED DESCRIPTION OF THE PREFERRED EMBODIMENTS

The anti-tryptase antibody of the present invention can be obtained by immunizing animal with tryptase and by preparing sera from the animal. The tryptase used herein is a new protease of trypsin type having a molecular weight of about 140,000 composed of four subunits with each a molecular weight of about 35,000. Such tryptase may be isolated from human lung mast cells, rat peritoneal mast cells, etc. [Kido et al., J. Biol. Chem., 256, 11939 (1981); Kido et al., Arch. Biochem. Biophys. 239, 436 (1985)]. Furthermore, as will be shown in Example 1, tryptase may also be obtained from mast cells of rat tongue or the like. Tryptase may be isolated from the homogenate of, for example, mast cells of the tongue, peritoneum, lung, etc. with appropriate combination of column chromatography such as S-Sepharose, arginine Sepharose, aprotinin Sepharose, etc. The tryptase referred to herein is not particularly limited but may be obtained from any animal. Alternatively, the tryptase may also be obtained by recombinant DNA technology.

For immunization with tryptase, rabbits, mice, sheep, horses, etc. may be used. Upon immunization, tryptase and Fruend's complete adjuvant are administered intradermally several times at a definite interval. After immunization, sera are prepared and subjected to, e.g., ammonium sulfate fractionation, DE52 column chromatography, etc. to obtain IgG fraction. Thus, the antitryptase antibody may be prepared. Alternatively, the IgG fraction may also be obtained by preparing the gamma globulin-containing fraction from sera by the ethanol fractionation [Cohn et al., J. Am. Chem. Soc., 68, 459 (1946)] and then precipitating with polyethylene glycol, ethanol, etc. [Schultze et al., "Molecular Biology of Human Proteins", Elsevier, Amsterdam, 2, 256 (1966)].

The anti-tryptase antibody of the present invention may be a monoclonal antibody. Such a monoclonal antibody may be prepared in a conventional method known as a method of Kohler and Milstein [Kohler et al., Nature, 256, 495 (1975)]. That is, animal is immunized with tryptase and then spleen cells are prepared.

The spleen cells are fused with myeloma cells to obtain hybridoma. By culturing the hybridoma in vivo or in vitro, the monoclonal antibody may be obtained.

In order to reduce further the antigenicity of the monoclonal antibody, there may also be used chimera antibody prepared by known methods in which the variable region from mouse antibody and the constant region from human antibody are fused [Sahagan et al., J. Immunology, 137, 1066 (1986); Nishimura et al., Nature, 332, 323 (1988)].

The anti-tryptase antibody of the present invention may be a fragment thereof containing its antigenbinding site. The antibody may also be used in the form of F(ab')2 fragment obtained by digesting the antibody with, e.g., pepsin [Nisonoff et al., Arch. Biochem. Biophys., 89, 230 (1960); Ishizuka et al., J. Immunology, 120, 800 (1978)] or in the form of Fab fragment obtained by digesting the antibody with papain [R.R. Porter, Biochemical Journal, 73, 119 (1959)].

It has been revealed by the present inventor that the anti-tryptase antibody or its fragment significantly inhibits HIV infection. That is, when MOLT-4 cells (ATCC CRL-1582) which are human acute lymphoblastic leukemia cells and CEM/LAV-1 cells obtained by persistent infection of CCRF-CEM cells (ATCC CCL-119) with LAV-1 (one of HIV) [Barre-Sinoussi et al., Science, 220, 868 (1983)] are co-cultured, syncytia are formed. It has been confirmed that in the case that the anti-tryptase antibody or its fragment is added to this system, the formation of syncytia is significantly inhibited and it was therefore indicated that the anti-tryptase antibody or its fragment significantly inhibits HIV infection.

Thus, the anti-tryptase antibody or its fragment according to the present invention is highly useful as drugs for the treatment of AIDS.

The anti-tryptase antibody or its fragment may be generally administered to patients with AIDS parenterally, e.g., intravenously, intramuscularly, subcutaneously, intrarectally, etc. Parenteral preparations include injections. The injections may be prepared in a conventional manner, for example, by subjecting the IgG fraction obtained as described above or its fragment to dialysis or sterile filtration, etc., if necessary and desired, and then dissolving or dispersing in an aqueous medium. In order to purify, the IgG fraction or its fragment may be further purified using an affinity column, onto which tryptase has been adsorbed. The injections may also contain mannitol, lactose, albumin, etc. In addition, preservatives, stabilizers, antiseptics, surface active agents which are conventionally used may also be incorporated into the injections, if necessary and desired.

A dose of the anti-tryptase antibody or its fragment may vary depending upon age, bodyweight or conditions of patients, etc. but may be generally in the range of from 20 to 2,000 mg/kg-body weight/day, preferably 50 to 1,000 mg/kg-body weight/day.

As is clear from the foregoing detailed descriptions, the anti-tryptase antibody can be produced in vivo by immunizing animal with tryptase, and the anti-tryptase antibody significantly inhibits HIV infection. From such facts, it will be apparent to one skilled in the art that tryptase itself may be used as a peptide vaccine for AIDS.

In the case of using tryptase as the vaccine, it is desired to use tryptase derived from animal such as rat, etc. from the aspect of its antigenicity.

In the vaccine preparations of tryptase, tryptase per se may be used alone or in combination with adjuvant. Examples of such adjuvant include aluminum hydroxide, aluminum phosphate, calcium phosphate or derivatives of muramyl dipeptide which is a constituent of the cell wall of tubercle bacillus, and the like.

Tryptase as the vaccine may be generally administered parenterally, e.g., intravenously, intramuscularly, subcutaneously, intrarectally, etc., as in the case of the anti-tryptase antibody. A dose of the vaccine is generally in the range of from 0.2 to 20 μg/kg-body weight, preferably 1 to 10 μg/kg-body weight, per administration, as tryptase.

The anti-tryptase antibody which can bind to tryptase specifically has a significant activity to inhibit HIV infection and is thus highly effective as a drug for the treatment of AIDS. Tryptase may be used as a vaccine for AIDS since tryptase induces production of the anti-tryptase antibody in vivo and the anti-tryptase antibody significantly inhibits HIV infection.

The present invention will be described in more detail by referring to test examples and examples but is not deemed to be limited thereto.

Example 1

Preparation of anti-tryptase antibody and its fragment

4

I. Preparation of tryptase

Tryptase was prepared by the method of Kido et al. [Kido et al., Arch. Biochem. Biophys. 239, 436 (1985)].

To rat tongue was added 20-fold amount of 0.1 M potassium phosphate buffer (pH 8.0) and the mixture was homogenized. After centrifugation, acetic acid was added to the supernatant to adjust to pH 4.5. The precipitates were removed by centrifugation and ammonium sulfate was added to the supernatant. The precipitates collected by centrifugation were dialyzed against 50 mM acetate buffer (pH 4.5), and applied to S-Sepharose column (manufactured by Pharmacia Fine Chemicals Inc.), which was previously equilibrated with 50 mM acetate buffer (pH 4.5). The column was washed with the same buffer and tryptase was eluted with a linear gradient of 0 - 1M NaCl. The active fraction was applied to Blue Sepharose column (manufactured by Pharmacia Fine Chemicals Inc.), which was then eluted with a linear gradient of 0 - 1M NaCl in 20 mM acetate buffer (pH 4.5). pH of the active fraction was adjusted to 6.5, and $CaCl_2$ was added to a final concentration of 10 mM. Then the mixture was subjected to Arginine Sepharose column (manufactured by Pharmacia Fine Chemicals Inc.), which was eluted with a linear gradient of 0 - 1 M NaCl in 50 mM Tris-HCl (pH 6.5) containing 10 mM $CaCl_2$. After the active fraction was concentrated by ultrafiltration, tryptase was further purified by liquid chromatography on TSK G3000SW column (manufactured by Toyo Soda Mfg. Co., Ltd.).

From the eluted positions in the liquid chromatography and the patterns of SDS-polyacrylamide gel electrophoresis [Laemmli, Nature, 227, 680 (1970)], the rat tongue-derived tryptase was considered to have four subunits each having a molecular weight of about 35,000.

II. Preparation of anti-tryptase antibody

1. Preparation of antisera

Tryptase (100 µg, 0.1 ml) obtained by the method described above were extensively mixed with 0.5 ml of Freund's complete adjuvant and 0.15 ml of the mixture was administered intradermally to four pads of a rabbit. Three weeks later, 60 µg of tryptase was administered to the same rabbit in a similar manner. After further 3 weeks, 60 µg of tryptase was administered to the same rabbit in a similar manner. Blood was collected four days after the final booster and sera were prepared.

2. Preparation of anti-tryptase IgG

To the rabbit anti-tryptase sera obtained in 1, was added 4-fold amount of PBS (50 mM phosphate buffer (pH 7.2) containing 0.1 M NaCl). An equal volume of 36% sodium sulfate was added to the mixture and stirred at room temperature for 30 minutes. After centrifugation at 12,000 x g for 20 minutes, the collected precipitates were suspended in a small volume of 50 mM phosphate buffer (pH 7.6) containing 0.15 M NaCl. The suspension was passed through ULTROGEL AcA34 (manufactured by LKB). Eluted fractions containing IgG were collected and dialyzed against 10 mM Tris-HCl (pH 8.0) at 4°C overnight. Then the sample was subjected to DE52 column (manufactured by Whatman Co., Ltd.) previously equilibrated with 10 mM Tris-HCl (pH 8.0). After extensively washing with the same buffer, IgG was eluted with a linear gradient of 0 -100 mM NaCl in 10 mM Tris-HCl (pH 8.0). IgG-containing fractions were collected and concentrated by ultrafiltration. It was confirmed that IgG was contained in this fraction by SDS-polyacrylamide gel electrophoresis [Laemmli, Nature, 227, 680 (1970)]. The concentration of the antibody was 527 µg/ml.

III. Property of anti-tryptase IgG

1. Confirmation of reactivity of anti-tryptase IgG with tryptase by Ouchterlony's method

It was confirmed by the Ouchterlony's method that the purified anti-tryptase IgG obtained in II. 2 had reacted with tryptase [Ouchterlony, Progr. Allergy, VI, 30 (1962)]. Agarose was dissolved in PBS at a concentration of 1% (w/v) and poured on a glass plate. Then, wells were made with a puncher and the antibody and the purified tryptase were dispensed therein. After several hours at room temperature sedimentation line could be observed between the anti-tryptase IgG and the purified tryptase. No sedimentation line could be observed between IgG purified from non-immunized rabbit sera and the purified tryptase. Furthermore, the anti-tryptase IgG did not react with trypsin derived from pancreas of bovine or rat.

## 2. Confirmation of reactivity of anti-tryptase IgG with tryptase by Western blotting

It was also confirmed by the Western blotting that the purified anti-tryptase IgG obtained in II. 2 had reacted with tryptase [Towbin et al., Proc. Natl.Acad. Sci. USA, 76, 4350 (1976)]. The purified tryptase was fractioned by SDS-polyacrylamide gel electrophoresis and transferred electrophoretically onto a nitro cellulose filter in 25 mM Tris-HCl/192 mM glycine (pH 8.3)/20% methanol. After blocking with l0% FCS in TBS (50 mM Tris-HCl (pH 8.0)/150 mM NaCl) at room temperature overnight, the filter was treated with anti-tryptase IgG diluted with TBS/1% BSA (bovine serum albumin) at room temperature for 5 hours. After washing 5 times with TBS/0.05% Tween 20 at room temperature for 5 minutes, a band of antibody-bound protein was detected by Pico Blue Immunodetection Kit (manufactured by STRATAGENE Co., Ltd). The results indicated that the anti-tryptase IgG had reacted with the purified tryptase. The anti-tryptase IgG did not react with trypsin derived from pancrease of bovine or rat.

## IV. Preparation of F(ab')$_2$ from the anti-tryptase antibody

The anti-tryptase IgG was cleaved with pepsin to prepare F(ab')$_2$ [Nisonofff et al., Arch. Biochem. Biophys., 89, 230 (1960); Mandy et al., J. Biol. Chem., 238, 206 (1963)]. To the anti-tryptase IgG was added 1/50 amount of pepsin. The mixture was incubated in 0.1 M acetate buffer (pH 4.5) at $37°$ C for 18 hours. After the pH was adjusted to 8.0 by dropwise addition of 1 N NaOH, the reaction mixture was centrifuged to remove insoluble matterials. Sodium sulfate was added to the supernatant at a final concentration of 0.18 g/ml. The precipitates were dissolved in 50 mM Tris-HCl (pH 8.0)/150 mM NaCl. The solution was passed through Sephadex G-150* (manufactured by Pharmacia Fine Chemicals Inc.) previously equilibrated with the same buffer, and F(ab')$_2$-containing fractions were collected. It was confirmed by the same method in III. 1 that this F(ab')$_2$ could react with tryptase like the anti-tryptase IgG.

Test Examples

## Test on inhibition of HIV infection

### 1. Cells used

Human acute lymphoblastic leukemia cells, i.e., MOLT-4 cells (ATCC CRL-1582), and CEM/LAV cells obtained by persistent infection of CCRF-CEM cells (ATCC CCL-119) with LAV-1, which is one of HIV [Barre-Sinoussi et al., Science, 220, 868 (1983)], were used.

### 2. Incubation of cells

The cells were cultured in RPMI 1640 medium supplemented with 10% FCS under conditions of $37°$ C, 5% $CO_2$ and relative humidity of 95%.

### 3. Test on inhibition of the syncytia formation

(1) Method

6

Test on inhibition of syncytia formation was performed according to the method of Lifson et al. [Lifson et al., J. Exp. Med., 164, 2101 (1986)]. Briefly, a part of the culture of MOLT-4 cells was centrifuged at 2,000 rpm for 5 minutes to collect the cells. After removing the supernatant, the cells were suspended in ASF 104 medium (manufactured by Ajinomoto Co., Ltd.) to prepare a cell suspension ($5 \times 10^5$ cells/ml). To this suspension various concentrations of anti-tryptase IgG, or F(ab')$_2$ of the anti-tryptase IgG was added. Each suspension was dispensed into a 96-well microtiter plate ($1 \times 10^5$ cells/well) and allowed to stand at $37°C$ for 30 minutes. The antibodies had been diluted previously with ASF 104 medium (manufactured by Ajinomoto Co., Ltd.) followed by dialysis against the same medium at $4°C$ overnight. Two microliter of CEM/LAV-1 cells ($1 \times 10^7$ cell/ml) was added to each well and then extensively mixed. After culturing at $37°C$ for 12 hours in the presence of 5% $CO_2$, syncytia formation was examined under an inverted microscope (magnification x 200). More than 60% inhibition of syncytia formation was evaluated as + +; more than 30 to 60% inhibition was evaluated as +; and 30% or less inhibition was evaluated as ±; and no syncytia formation was evaluated as -. Thus, the inhibiting activity of syncytia formation was evaluated.

(2) Results

The results of the evaluation for the ability to inhibit the syncytia formation are shown in the following table.

| Ability of inhibiting syncytium formation of Anti-tryptase IgG | | | |
|---|---|---|---|
| Dilution fold of anit-tryptase IgG | $1/3^0$ | $1/3^1$ | $1/3^2$ |
| Inhibition of syncytia formation | + + | + + | - |

As is clear from the above, the anti-tryptase IgG significantly inhibits the syncytia formation depending on its concentration. In the case of F(ab')$_2$ fragment of the anti-tryptase IgG, inhibition of the syncytia formation was also observed to an extent similar to the case with the anti-tryptase IgG. However, IgG purified from the non-immunized rat sera did not inhibit the syncytia formation.

Example 2 Injections of anti-tryptase antibody:

1. Preparation of affinity column

To 1 g of activated CH-Sepharose 4B (manufactured by Pharmacia Fine Chemicals Inc.) was added 1 mM HCl to swell the gel. The gel was then washed with 1 mM HCl. The purified tryptase (200 µg) (dissolved in 0.1 M NaHCO$_3$ (pH 8.0)) was added thereto and mixed with the swollen gel at room temperature for an hour. The gel was washed with 50 mM Tris-HCl (pH 8.0)/0.5 M NaCl, then with 50 mM sodium acetate (pH 4.0)/0.5 M NaCl. The washing was repeated 3 times.

2. Purification of anti-tryptase IgG using affinity column

The anti-tryptase IgG prepared in Example 1, II. 2 was applied to the affinity column prepared in 1. above. After washing, the anti-tryptase IgG was eluted with 0.1 M phosphate buffer (pH 10.8). After pH was immediately adjusted to 7.5, the eluate was concentrated by a ultrafiltration and dialyzed. The IgG was freeze-dried and then dissolved in sterile water to prepare l6% solution.
The activity of the thus purified anti-tryptase IgG for inhibiting the syncytia formation was assayed by the same method in Test Example. The activity was significantly recognized.

3. Preparation of injections of anti-tryptase antibody

Using the anti-tryptase IgG purified in 2. above, injections were prepared in a conventional method in

7

which glycine was used as a stabilizer and methyl thiolate was used as an antiseptic.

The injections may be intramuscularly administered in a dose of from 20 to 2,000 mg/kg-body weight/day, preferably 50 to 1,000 mg/kg-body weight/day, as IgG or its fragment.

Example 3 Preparation of anti-AIDS vaccine preparations

The purified rat tryptase in Example 1, I was dialyzed against water and then dried to prepare vaccines for AIDS.

The preparations are dissolved in physiological saline upon use and the solution may be subcutaneously injected. The dose may be in the range of from 0.2 to 20 $\mu$g/kg-body weight, preferably 1 to 10 $\mu$g/kg-body weight, per administration as tryptase.

The solution was administered to rabbit by the same method in Example 1, II. 1 and evaluated by the same method in Example 1, II. 2. Production of the antibody was confirmed.

**Claims**

1. An anti-tryptase antibody, or a fragment thereof, capable of specifically reacting with tryptase.

2. An anti-tryptase antibody according to claim 1, wherein said tryptase is a trypsin type protease having a molecular weight of about 140,000 and which is composed of four subunits, each of which has a molecular weight of about 35,000.

3. An anti-tryptase antibody according to Claim 1 or 2 wherein said antibody is an IgG, or IgG F(ab')$_2$ or Fab fragment.

4. A pharmaceutical composition comprising as an active ingredient tryptase inhibitor or an antibody or fragment according to any preceding Claim together with a pharmaceutically acceptable carrier therefor.

5. A pharmaceutical composition according to Claim 4, for the treatment of AIDS.

6. A vaccine for AIDS comprising a tryptase.

7. A vaccine according to Claim 6, wherein said tryptase is a trypsin type protease having a molecular weight of 140,000 and which is composed of four subunits, each of which has a molecular weight of about 35,000.

8. A vaccine according to Claim 6 or 7, further comprising an adjuvant.

9. Use of a tryptase inhibitor or an antibody or fragment according to any of Claims 1 to 3 for the manufacture of a medicament for the treatment or prophylaxis of AIDS.